# EUROPÄISCHE PATENTANMELDUNG

(11) **EP 0 931 565 A2**
(43) Veröffentlichungstag der Anmeldung: **28.07.1999**
(21) Anmeldenummer: 98122663.2
(22) Anmeldetag: 30.11.1998
(51) Int. Cl.: A61N 1/34

(54) **Bandage, insbesondere Halskause, Rückenbandage, Ellenbogenbandage und Kniebandage**

(30) Priorität: 19.01.1998 DE 29800675 U
(71) Anmelder: Schnittker, Franz-Joseph Dr., 32657 Lemgo (DE)
(72) Erfinder: Schnittker, Franz-Joseph Dr., 32657 Lemgo (DE)
(74) Vertreter: Eikel, Cordula

(57) **Zusammenfassung**

Eine Bandage, insbesondere Halskrause, Rückenbandage, Ellenbogenbandage und Kniebandage ist dadurch gekennzeichnet, daß sie mit einer Einrichtung zur transkutanen Nervenstimulation, insbesondere zur Schmerzbehandlung versehen ist, so daß eine Bandage geschaffen wird, bei dem gleichzeitig, d.h. kombiniert, die entsprechenden Partien auch einer Reiz- bzw. Elektrotherapie, d.h. z.B. einer transkutanen Nervenstimulation unterzogen werden können, ohne daß dazu Elektroden gesondert auf der Haut plaziert und fixiert werden müssen, so daß eine Kombination von Bandage und Nervenstimulation ohne gesonderte Positionierung von Elektroden vorgenommen werden kann.

## Beschreibung

Die Erfindung betrifft eine Bandage, insbesondere Halskrause, Rückenbandage, Ellenbogenbandage und Kniebandage mit den Merkmalen des Oberbegriffes des Schutzanspruches 1.

Zur Schmerzbehandlung wird häufig eine transkutane Nervenstimulation zur Anwendung gebracht, bei der Elektroden im Bereich der zu stimulierenden Nerven auf der Haut angebracht werden, um dann eine Nervenstimulation mittels Strom vorzunehmen, wobei die Stromstärke z.B. 20 bis 200 Herz betragen kann und insgesamt regelbar ist.

Eine derartige Behandlung macht es jedoch erforderlich, daß die Elektroden gesondert in den zu stimulierenden Bereichen angebracht werden müssen, um dann mit den entsprechenden Apparaturen, d.h. Stromquellen, versehen zu werden.

Elektroden werden dabei z.B. mittels Pflaster oder auch selbstklebend auf der Haut angebracht.

Der Erfindung liegt die Aufgabe zugrunde, eine derartige transkutane Nervenstimulation vornehmen zu können, ohne daß der zu Behandelnde jeweils Elektroden unmittelbar auf der Haut festzulegen hat, sei es durch z.B. Pflaster oder selbstklebende Elektroden.

Diese Aufgabe wird durch eine Bandage, insbesondere Halskrause, Rückenbandage, Ellenbogenbandage und Kniebandage mit den Merkmalen des kennzeichnenden Teiles des Schutzanspruches 1 gelöst. Weitere vorteilhafte Ausgestaltungen der Erfindung sind Gegenstand der Unteransprüche

Dadurch, daß die Bandage mit einer Einrichtung zur transkutanen Nervenstimulation, insbesondere zur Schmerzbehandlung versehen ist, ist es möglich, eine derartige Stimulation während des Tragens der Bandage vorzunehmen, d.h. die behandelnde Person kann gleichzeitig sowohl den gewünschten Behandlungseffekt durch die zu tragende Bandage als auch durch die transkutane Nervenstimulation erzielen, ohne daß er dazu während der z.B. transkutanen Nervenstimulation an einem entsprechenden Gerät zu verweilen hat oder aber sich in ambulante Behandlung zu begeben hat, vielmehr besitzt der zu Behandelnde, trotz der Anwendung durch die Einrichtung, d.h. der transkutanen Nervenstimulation, weitgehende Bewegungsfreiheit, d.h. eingeschränkt lediglich durch das Tragen der erfindungsgemäßen Bandage.

Durch die erfindungsgemäße Bandage wird eine Kombination von z.B. Kompression und transkutaner Nervenstimulation erreicht, sei es im Halswirbel- oder auch z.B. im Rückenbereich bei z.B. notwendiger Stabilisierung. Hinzu kommt, daß die Elektroden nicht jeweils gesondert auf der Haut zu fixieren und damit zu plazieren sind, sondern daß diese bei entsprechendem Anlegen der Bandage in der richtigen Position ohne weitere Positionierungsnotwendigkeiten angeordnet sind.

Weist die Einrichtung eine Steuereinrichtung auf, so ermöglicht dies dem Anwender eine entsprechende Steuerung der Nervenstimulation.

Ist die Steuereinheit an oder in der Bandage angeordnet bzw. integriert, so ermöglicht auch dies eine größtmögliche Bewegungsfreiheit.

Ist die Steuereinheit separat von der Bandage anzuordnen, so ermöglicht dies auch den Einsatz einer Einrichtung zur transkutanen Nervenstimulation bei z.B. Halskrausen ohne großen konstruktiven Aufwand.

Weist die Bandage sowohl die Einrichtung zur transkutanen Nervenstimulation als auch eine Einrichtung zur Wärmebehandlung und/oder eine Einrichtung zur sonstigen Stimulation auf, so ermöglicht dies verschiedene Behandlungen durch Tragen der Bandage, ohne daß es dazu jeweils gesonderter Einrichtungen bzw. Behandlungsabschnitte bedarf.

Die erfindungsgemäße Bandage wird anhand der folgenden Ausführungsbeispiele näher erläutert.

Bei der erfindungsgemäßen Bandage kann es sich z.B. um eine Halskrause oder auch um eine Cervicalstütze handeln.

Bei einer derartigen Halskrause wird zur Schmerzbehandlung eine Vorrichtung zur transkutanen Nervenstimulation integriert, wobei die mit der Körperhaut in Kontakt gelangenden Elektroden auf der Halskrauseninnenseite angeordnet sein können oder aber Teil der Halskrauseninnenbeschichtung sein können, d.h. es ist denkbar, daß die Halskrauseninnenseite Gewebeabschnitte aufweist, die als Elektroden fungieren, sei es durch eingewebtes Material oder durch herkömmliche integrierte Elektroden.

Die dazugehörigen Leitungen, d.h. Stromverbindungen, sind dabei in der Halskrause integriert.

Die Halskrause weist Anschlüsse für die Steuereinheit auf, die gesondert z.B. im Bereich des Hosenbundes zu plazieren ist und über ein entsprechendes Verbindungskabel mit der Halskrause in Wirkungseingriff bzw. Steuereingriff steht.

Es ist jedoch auch denkbar, daß die Steuereinheit kabellos mit den Elektroden in Verbindung steht und die Energiequellen in der Halskrause integriert ist.

Es ist in einem weiteren Ausführungsbeispiel ebenso denkbar, die Halskrause und/oder Cervicalstütze nicht nur mit einer Vorrichtung zur transkutanen Nervenstimulation zur Schmerzbehandlung auszustatten, sondern auch mit anderen Vorrichtungen wie z.B. einer Wärmequelle, die ebenfalls über ein externes Steuergerät ansteuerbar sein kann oder sonstige Stimulationseinrichtungen.

Dabei kann die Steuereinheit für die transkutane Nervenstimulation sowie die Steuereinheit für die Wärmebehandlung oder sonstige Stimulation für z.B. im hier vorliegenden Ausführungsbeispiel die Nackenmuskulatur kombiniert sein, d.h. sowohl der Ansteuerung der transkutanen Nervenstimulation als auch sonstiger Stimulation und/oder der Wärmebehandlung dienen.

Ebenso wäre es denkbar, jeweils gesonderte Steuereinheiten vorzusehen.

Bei z.B. Rückenbandagen ist es ebenso denkbar, eine entsprechende Einrichtung zur transkutanen Nervenstimulation zur Schmerzbehandlung in die Rückenbandage zu integrieren, wobei auch hier entsprechende herkömmliche Elektroden auf, an oder in der Rückenbandage angeordnet werden können.

Es ist dabei ebenso denkbar, daß die, dem Körper zuzuwendende Seite der z.B. Rückenbandage Gewebeabschnitte aufweist, die als Elektrode fungieren oder aber derartig leitfähig ausgestaltet sind, daß unter ihr angeordnete Elektroden, die beabsichtigten Stimulationen, d.h. Stromstimulationen an die Körperhaut abgeben können.

Auch hier ist es denkbar, die Steuereinheit für die transkutane Nervenstimulation gesondert von der Bandage anzuordnen oder aber integriert, d.h. in der Bandage selbst anzuordnen, so daß eine Steuerung unmittelbar über die Bandage bzw. die dort angeordnete Steuereinheit vorgenommen werden kann, d.h. eine gesonderte Plazierung durch den Patienten nicht erforderlich ist.

Dabei kann die Steuereinheit selbstverständlich der Art gestaltet sein, daß die transkutane Nervenstimulation vom Patienten nur an- oder ausgestellt werden kann, d.h. vom Fachpersonal bereits eine entsprechende Stärke vorbestimmt ist oder aber verschiedene Steuereinheiten für verschiedene z.B. Stromstärken vorgesehen sind.

Auch hier ist eine Kombination mit verschiedenen Behandlungsmöglichkeiten denkbar, d.h. z.B. ist es denkbar, daß auch die Rückenbandage sowohl eine Heizeinrichtung als auch eine Einrichtung zur transkutanen Nervenstimulation und/oder weitere Stimulationseinrichtungen beinhaltet.

Das zuvorgesagte gilt selbstverständlich auch für jedwede andere Bandage, wie auch Halskrausen oder sonstige am Körper anliegende oder anlegbare Einrichtungen.

Selbstverständlich ist es auch bei derartigen Bandagen denkbar, daß das Gewebe der Bandage oder aber entsprechendes leitfähiges Material in Gänze oder abschnittsweise eingearbeitet, wie z.B. eingewebt ist, so daß es keiner gesonderten herkömmlichen Elektroden bedarf.

Die Steuereinheit kann dabei mittels Batterien oder auch Akkus betrieben werden, wobei eine Wiederaufladbarkeit selbstverständlich gewährleistet ist, d.h. entweder durch herkömmliches Aufladen von z.B. Akkus oder unmittelbares Anschließen der Steuereinheit an eine Stromquelle.

## Patentansprüche

1. Bandage, insbesondere Halskrause, Rückenbandage, Ellenbogenbandage und Kniebandage, **dadurch gekennzeichnet,** daß sie mit einer Einrichtung zur transkutanen Nervenstimulation, insbesondere zur Schmerzbehandlung versehen ist.

2. Bandage nach Anspruch 1, dadurch gekennzeichnet, daß die Einrichtung eine Steuereinrichtung aufweist.

3. Bandage nach Anspruch 2, dadurch gekennzeichnet, daß die Steuereinrichtung eine Steuereinheit ist und daß die Steuereinheit an oder in der Bandage integriert ist.

4. Bandage nach Anspruch 3, dadurch gekennzeichnet, daß die Steuereinheit separat von der Bandage anordnungsbar ausgestaltet ist.

5. Bandage nach einem der Ansprüche 1 bis 4, dadurch gekennzeichnet, daß die Einrichtung der Reizübertragung dienende Elektroden aufweist.

6. Bandage nach Anspruch 5, dadurch gekennzeichnet, daß die Elektroden auf der dem Körper zuzuwendenden Seite der Bandage anordnungsbar ausgestaltet sind.

7. Bandage nach Anspruch 6, dadurch gekennzeichnet, daß wenigstens auf der dem Körper zuzuwendenden Seite der Bandage Elektroden angeordnet sind.

8. Bandage nach Anspruch 5 oder 7, dadurch gekennzeichnet, daß die Elektroden in das Bandagengewebe bzw. -beschichtung, integriert sind.

9. Bandage nach Anspruch 5, 7 oder 8, dadurch gekennzeichnet, daß das Bandagengewebe bzw. - beschichtung wenigstens teilweise als Elektrode ausgestaltet ist.

10. Bandage nach einem der Ansprüche 5 bis 9, dadurch gekennzeichnet, daß die Elektroden in der Bandage angeordndet sind und daß das dem Körper zuzuwendende, die Elektroden abdeckende Bandagengewebe bzw. - beschichtung, zumindest im Bereich der Elektroden, die Reizübertragung ermöglichend ausgestaltet ist.

11. Bandage nach einem der Ansprüche 1 bis 10, dadurch gekennzeichnet, daß neben der Einrichtung zur transkutanen Nervenstimulation eine Einrichtung zur Wärmebehandlung vorgesehen ist.

12. Bandage nach einem der Ansprüche 1 bis 11, dadurch gekennzeichnet, daß eine Einrichtung zur transkutanen Nervenstimulation und/oder eine Einrichtung zur Wärmebehandlung und/oder eine Einrichtung zur sonstigen Stimulation vorgesehen ist.

13. Bandage nach einem der Ansprüche 1 bis 12, dadurch gekennzeichnet, daß die Einrichtung zur transkutanen Nervenstimulation und/oder die Einrichtung zur Wärmebehandlung und/oder die Einrichtung zur sonstigen Stimulation jeweils eine gesonderte Steuereinheit aufweist.

14. Bandage nach Anspruch 13, dadurch gekennzeichnet, daß die Einrichtung zur transkutanen Nervenstimulation und/oder die ggfls. vorhandene Einrichtung zur Wärmebehandlung und/oder die ggfls. vorhandene Einrichtung zur sonstigen Stimulation eine gemeinsame Steuereinheit aufweist.

15. Bandage nach einem der Ansprüche 1 bis 14, dadurch gekennzeichnet, daß die Steuereinheit zur transkutanen Nervenstimulation die Stromstärke und/oder Stromfrequenz ansteuerbar ausgestaltet ist und/oder eine Aus-/Einfunktion aufweist.
